# EUROPEAN PATENT SPECIFICATION

(11) **EP 0 874 864 B1**
(45) Date of publication and mention of the grant of the patent: **14.09.2005**
(21) Application number: 95944583.4
(22) Date of filing: 19.12.1995
(51) Int. Cl.: C07K 14/00, C07K 14/435, C07K 14/475, C12N 1/13, C12N 1/21, C12N 15/12, C12N 15/18, C12N 15/63

(54) **ANTIVIRAL PROTEIN**
ANTIVIRALES PROTEIN
PROTEINE ANTIVIRALE

(43) Date of publication of application: 04.11.1998
(73) Proprietor: HUMAN GENOME SCIENCES, INC., Rockville, MD 20850-3338 (US)
(72) Inventor: NI, Jian, Rockville, MD 20853 (US); FENG, Ping, Gaithersburg, MD 20878 (US); DILLON, Patrick, J., Gaithersburg, MD 20878 (US); GENTZ, Reiner, Silver Spring, MD 20904 (US)
(74) Representative: Vossius & Partner
(86) International application number: PCT/US1995/017107
(87) International publication number: WO 1997/022623

(56) References cited:
- JOURNAL OF BIOLOGICAL CHEMISTRY, Volume 270, issued 07 July 1995, J. BHALERAO et al., "Molecular Cloning, Characterization and Genetic Mapping of the cDNA Coding for a Novel Secretory Protein of Mouse", pages 16385-16394.
- DATABASE EBI [Online] H66471, 21 October 1995 WILSON R.K.: 'EST'

## Description

This invention relates to newly identified polynucleotides, polypeptides encoded by such polynucleotides, the use of such polynucleotides and polypeptides, as well as the production of such polynucleotides and polypeptides. The polypeptide of the present invention has been putatively identified as a human antiviral protein, and in particular as an osteo antiviral protein, sometimes hereinafter referred to as "OAP." The invention also relates to inhibiting the action of such polypeptides.

The process of embryonic bone formation involves the creation of an extracellular matrix that mineralizes during the course of tissue maturation. This matrix is subject to constant remodeling during the lifetime of an individual, through the combined actions of osteoblasts and osteoclasts. A careful balance of matrix formation and resorption must be maintained because perturbations can result in various bone disorders.

The extracellular matrix of bone consists of two phases, an organic phase and a mineral phase. The organic phase consists primarily of the collagen type I fibrils that are associated with a number of noncollagenous matrix proteins. Interest in the noncollagenous proteins of the bone has been greatly stimulated since Urist first demonstrated that demineralized bone extracts could induce ectopic bone formation (Urist, M.R., Science, 150:893-899 (1965)). Noncollagenous proteins of bone are now believed to be involved in mineralization as well as the local regulation of bone cell function (Heinegard, D. and Oldberg, A., Connective Tissue and Its Heritable Disorders (Royce, P.M. and Steinmann, B., EDS), pages 189-209, Wiley-Liss, New York (1993), and Von der Mark, K. and Goodman, S., id.). In the past few years, a number of noncollagenous proteins of bone have been isolated and characterized; among these are osteocalcin, osteopontin, osteonectin and bone sialoprotein (Heinegard, D. and Oldberg, A., FASEB J., 3:2042-2051 (1985)).

A clonal osteogenic cell line (MN7) from bone marrow stroma of the adult mouse has been established (Mathieu, E., *et al*., Calcif. Tissue Int., 50:362-371 (1992)). These cells, under appropriate conditions, undergo typical osteoblastic differentiation *in vitro* and are able to form a mineralized extracellular matrix (Mathieu, E. and Merregaert, J., J. Bone Miner. Res., 9:183-192 (1994)).

A cDNA coding for a novel secretory protein of mouse (p85), has been cloned, characterized and genetically mapped (Bhalerao, J., *et al*., J. Biol. Chem., 270 (27) :16385-16394 (1995)). The full-length cDNA contains an open reading frame of 1677 bp encoding a protein of 559 amino acids. The clone contains a hydrophobic signal peptide characteristic of a secreted protein. The message of 1.9 kb is expressed in various tissues, such as liver, heart, lungs, etc., whereas a splice variant was present in embryonic cartilage in skin. This gene p85, called Ecm1 for extracellular matrix protein 1, maps on chromosome 3 of mouse in a region containing several loci involved in skin development disorders.

The polypeptide of the present invention has highest amino acid sequence homology to a growth factor, Ecm1, and it has been shown to have anti-viral activity.

In accordance with one aspect of the present invention, there is provided a novel mature polypeptide, according to claim 10 as well as biologically active and diagnostically or therapeutically useful fragments, analogs and derivatives according to claim 10. The polypeptide of the present invention is of human origin.

In accordance with another aspect of the present invention, there are provided isolated nucleic acid molecules encoding a polypeptide of the present invention including mRNAs, cDNAs, genomic DNAs as well as analogs and biologically active and diagnostically or therapeutically useful fragments according to claim 1.

In accordance with another aspect of the present invention there is provided an isolated nucleic acid molecule encoding a mature polypeptide expressed by the DNA contained in ATCC Deposit No. 97302.

In accordance with yet a further aspect of the present invention, there is provided a process for producing such polypeptide according to claims 8 and 9 by recombinant techniques comprising culturing recombinant prokaryotic and/or eukaryotic host cells, containing a nucleic acid sequence encoding a polypeptide of the present invention, under conditions promoting expression of said protein and subsequent recovery of said protein.

In accordance with yet a further aspect of the present invention, there is provided a process for utilizing such polypeptide according to claim 16, or polynucleotide encoding such polypeptide for therapeutic purposes, for example, as an antiviral factor to treat disease caused by infection with virus.

In accordance with yet a further aspect of the present invention, there are provided antibodies against such polypeptides according to claim 11.

In accordance with another aspect of the present invention, there are provided OAP agonists which mimic OAP and bind to the OAP receptors and antagonists against such polypeptides according to claims 11-13, which may be used to inhibit the action of such polypeptides.

In accordance with still another aspect of the present invention, there are provided diagnostic assays for detecting diseases or susceptibility to diseases related to mutations in the nucleic acid sequences according to claims 17 and 18 encoding a polypeptide of the present invention.

In accordance with yet a further aspect of the present invention, there is provided a process for utilizing such polypeptides, or polynucleotides encoding such polypeptides according to claim 16, for *in vitro* purposes related to scientific research, for example, synthesis of DNA and manufacture of DNA vectors.

These and other aspects of the present invention should be apparent to those skilled in the art from the teachings herein.

The following drawings are illustrative of embodiments of the invention and are not meant to limit the scope of the invention as encompassed by the claims.
Figure 1 is an illustration of the cDNA and corresponding deduced amino acid sequence of the polypeptide of the present invention. The underlined portion is indicative of a putative leader sequence. Sequencing was performed using a 373 automated DNA sequencer (Applied Biosystems, Inc.).

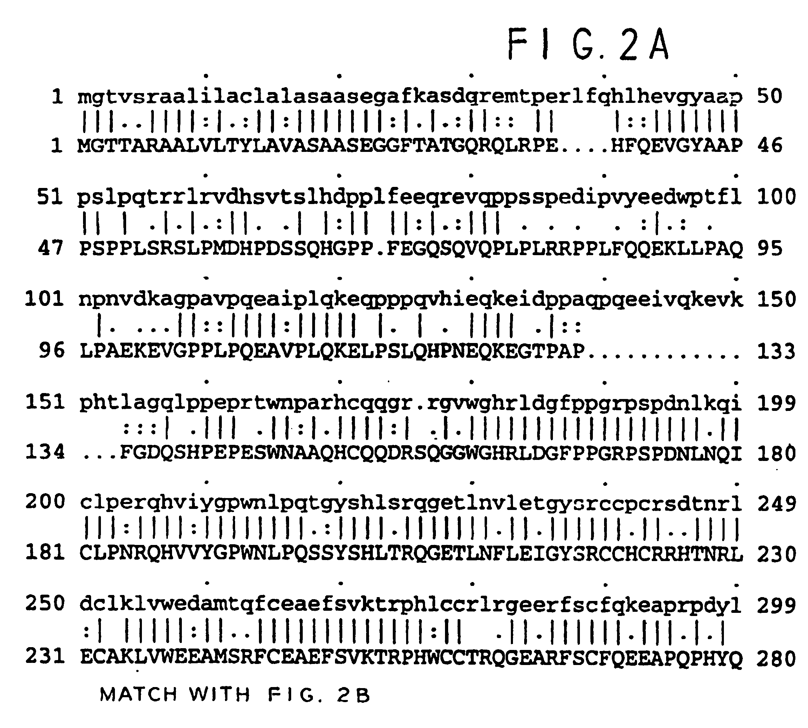

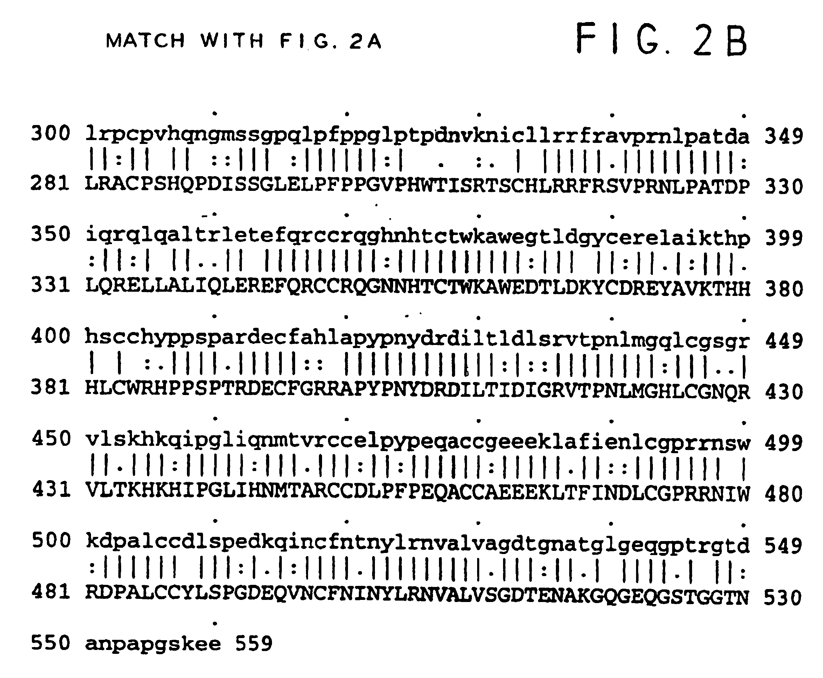

Figure 2 is an amino acid sequence comparison between the polypeptide of the present invention (top line) and murine Ecm1 (bottom line).
Figure 3 shows structural and functional features of OAP deduced by the indicated techniques, as a function of amino acid sequence.
Figure 4 is a graph showing that OAP protects mice from EMCV infection. As described in Example 4, groups of 10 eight week-old male A/J mice were injected with saline (mock), murine IFN-gamma, 43 ug OAP or 84 ug OAP, and then challenged with EMCV. Survivors in each group on successive days after exposure to EMCV are depicted in the graph.

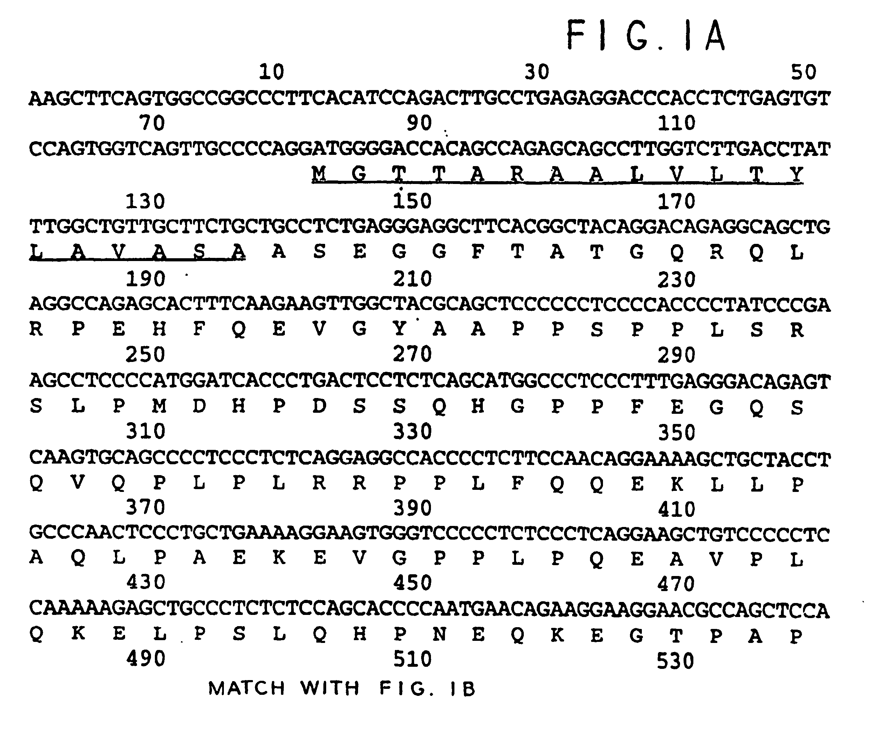

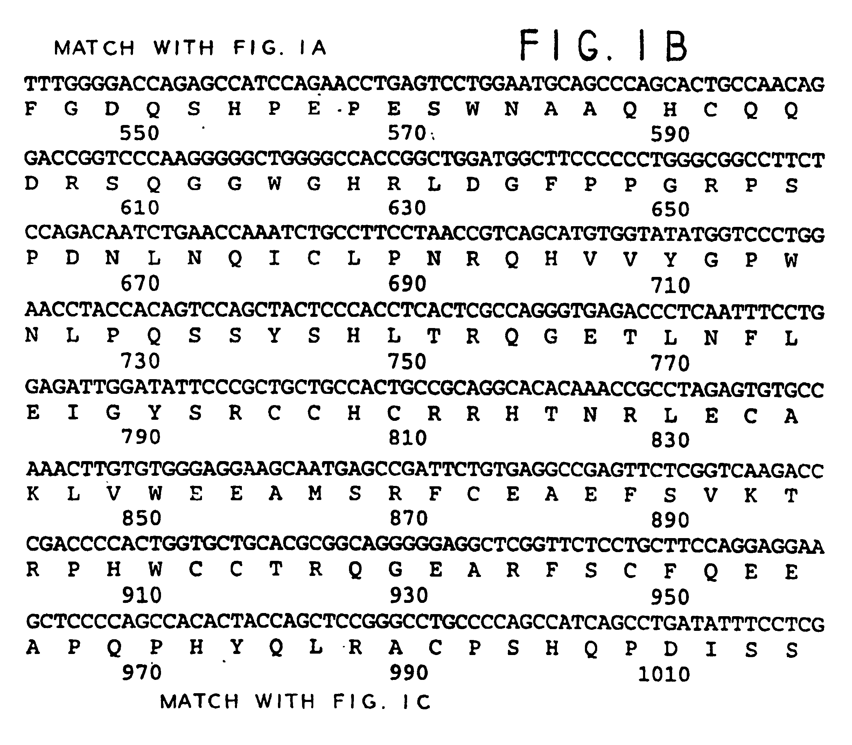

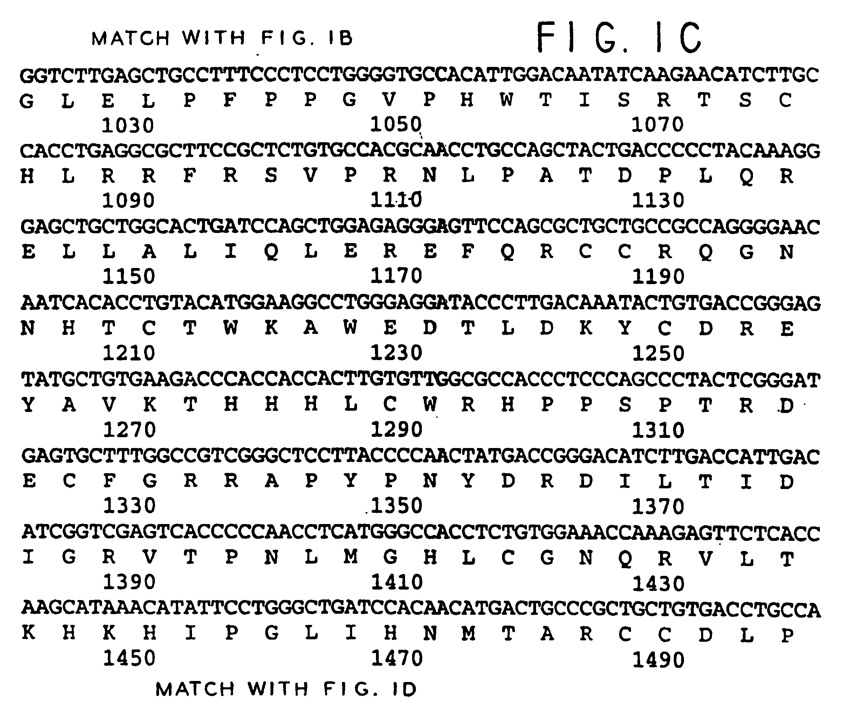

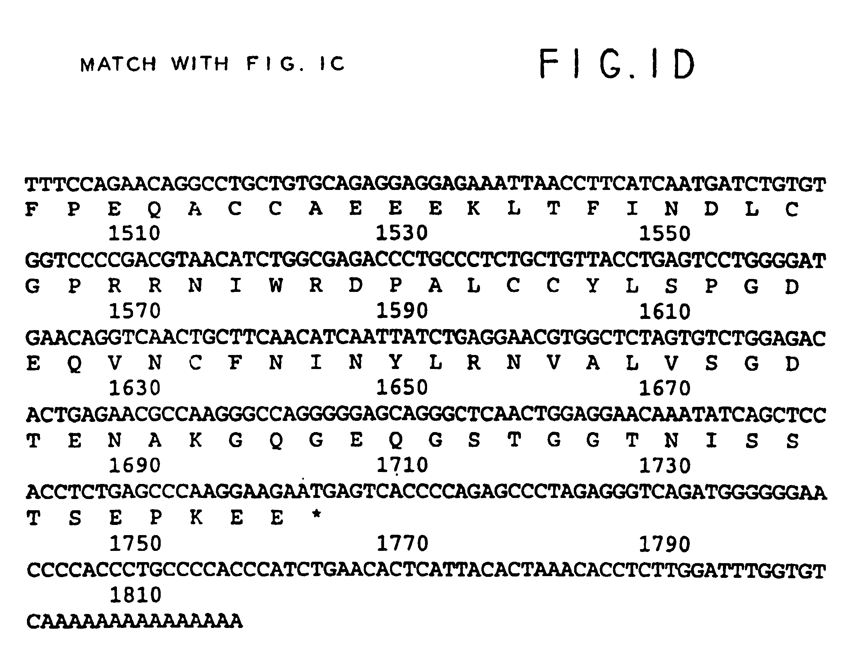

In accordance with an aspect of the present invention, there is provided an isolated nucleic acid (polynucleotide) which encodes for the mature polypeptide having the deduced amino acid sequence of Figure 1 (SEQ ID NO:2).

The polynucleotide of this invention was discovered in a cDNA library derived from a human tumor pancreas. It is structurally related to the Ecm1. It contains an open reading frame encoding a protein of 540 amino acid residues of which the first 19 amino acids residues are the putative leader sequence such that the mature protein comprises 521 amino acids. The protein exhibits the highest degree of homology to murine Ecm1 at the amino acid level with 66.790% identity and 78.664% similarity over the entire amino acid stretch. The gene of the present invention exhibits the highest degree of homology at the nucleotide level also to murine Ecm1 with 80% identity and 80% similarity over the entire nucleotide sequence. The mature OAP protein has a predicted molecular weight of 59182.10, a molar extinction coefficient of approximately 74220, and an isoelectric point of 6.80.

In accordance with another aspect of the present invention there are provided isolated polynucleotides encoding a mature polypeptide expressed by the DNA contained in ATCC Deposit No. 97302, deposited with the American Type Culture Collection, 12301 Park Lawn Drive, Rockville, Maryland 20852, USA, on September 25, 1995. The deposited material is a plasmid that contains the full-length OAP cDNA inserted into a pBluescript SK(-) vector (Stratagene, La Jolla, CA).

The deposit has been made under the terms of the Budapest Treaty on the International Recognition of the Deposit of Micro-organisms for purposes of Patent Procedure. The strain will be irrevocably and without restriction or condition released to the public upon the issuance of a patent. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are controlling in the event of any conflict with any description of sequences herein. A license may be required to make, use or sell the deposited materials, and no such license is hereby granted. References to "polynucleotides" throughout this specification includes the DNA of the deposit referred to above.

The polynucleotide of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. The coding sequence which encodes the mature polypeptide may be identical to the coding sequence shown in Figure 1 (SEQ ID NO:1) or may be a different coding sequence which coding sequence, as a result of the redundancy or degeneracy of the genetic code, encodes the same mature polypeptide as the DNA of Figure 1 (SEQ ID NO:1).

The polynucleotide which encodes for the mature polypeptide of Figure 1 (SEQ ID NO:2) includes the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 (SEQ ID NO:2) according to claim 1. The variant of the polynucleotide may be a naturally occurring allelic variant of the polynucleotide or a non-naturally occurring variant of the polynucleotide.

Thus, the present invention includes polynucleotides encoding the same mature polypeptide as shown in Figure 1 (SEQ ID NO:2) as well as variants according to claim 1 of such polynucleotides which variants encode for a fragment, derivative or analog of the polypeptide of Figure 1 (SEQ ID NO:2). Such nucleotide variants include deletion variants, substitution variants and addition or insertion variants.

As hereinabove indicated, the polynucleotide according to claim 1 may have a coding sequence which is a naturally occurring allelic variant of the coding sequence shown in Figure 1 (SEQ ID NO:1). As known in the art, an allelic variant is an alternate form of a polynucleotide sequence which may have a substitution, deletion or addition of one or more nucleotides, which does not substantially alter the function of the encoded polypeptide.

The present invention also includes polynucleotides according to claim 1 wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains. Thus, for example, the polynucleotide of the present invention may encode for a mature protein, or for a protein having a prosequence or for a protein having both a prosequence and a presequence (leader sequence).

The polynucleotides according to claim 1 may also have the coding sequence fused in frame to a marker sequence which allows for purification of the polypeptide of the present invention. The marker sequence may be a hexa-histidine tag supplied by a pQB-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The term "gene" means the segment of DNA involved in producing a polypeptide chain; it includes regions preceding and following the coding region (leader and trailer) as well as intervening sequences (introns) between individual coding segments (exons).

Fragments of the full length gene of the present invention may be used as a hybridization probe for a cDNA library to isolate the full length cDNA and to isolate other cDNAs which have a high sequence similarity to the gene or similar biological activity. Probes of this type preferably have at least 30 bases and may contain, for example, 50 or more bases. The probe may also be used to identify a cDNA clone corresponding to a full length transcript and a genomic clone or clones that contain the complete gene including regulatory and promotor regions, exons, and introns. An example of a screen comprises isolating the coding region of the gene by using the known DNA sequence to synthesize an oligonucleotide probe. Labeled oligonucleotides having a sequence complementary to that of the gene of the present invention are used to screen a library of human cDNA, genomic DNA or mRNA to determine which members of the library the probe hybridizes to.

The present invention further relates to polynucleotides which hybridize to the hereinabove-described sequences if there is at least 85%, preferably at least 90%, and more preferably at least 95% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which either retain substantially the same biological function or activity as the mature polypeptide encoded by the cDNAs of Figure 1 (SEQ ID NO:1).

Thus, the present invention is directed to polynucleotides having at least an 85% identity, preferably at least a 90% identity and more preferably at least a 95% identity to a polynucleotide which encodes the polypeptide of SEQ ID NO:2 and polynucleotides complementary thereto.

The present invention further relates to a polypeptide which has the deduced amino acid sequence of Figure 1 (SEQ ID NO:2), as well as fragments, analogs and derivatives of such polypeptide according to claim 1.

The terms "fragment," "derivative" and "analog" when referring to the polypeptide of Figure 1 (SEQ ID NO:2), means a polypeptide which retains essentially the same biological function or activity as such polypeptide. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 (SEQ ID NO:2) may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

Also preferred in this aspect of the invention are fragments characterized by structural or functional attributes of OAP. Preferred embodiments of the invention in this regard include fragments that comprise alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of OAP.

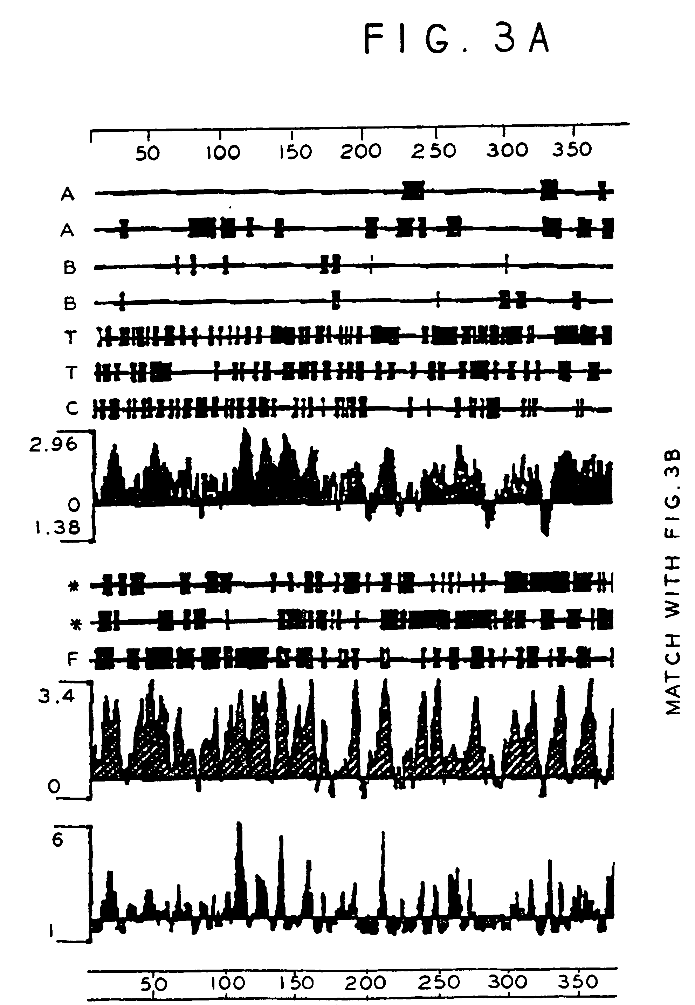

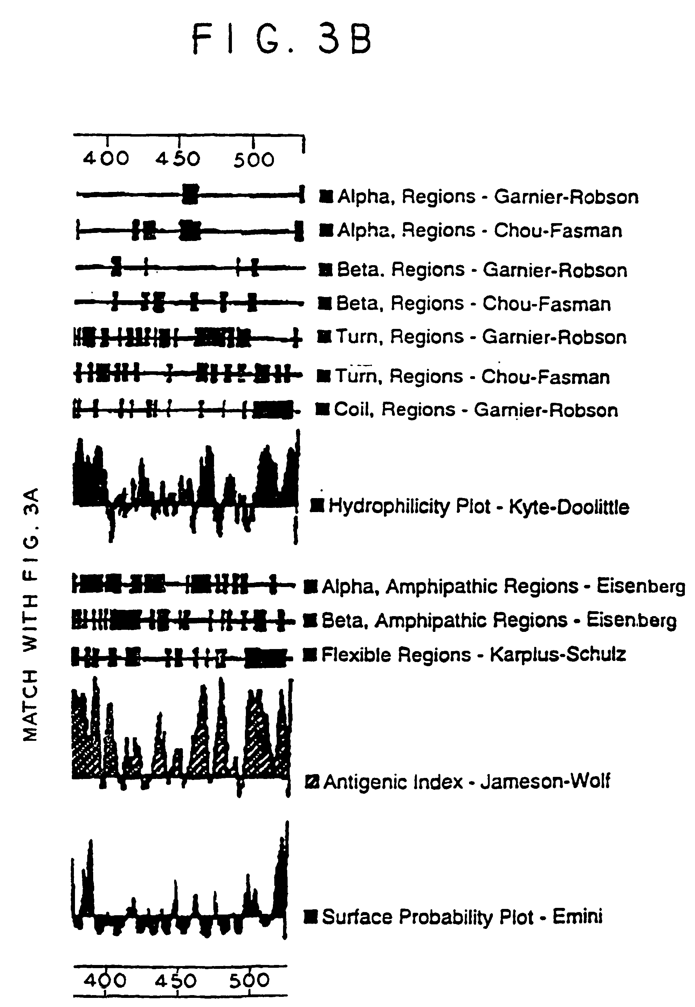

Certain preferred regions in these regards are set out in Figure 3, and include, but are not limited to, regions of the aforementioned types identified by analysis of the amino acid sequence set out in Figure 1. As set out in Figure 3, such preferred regions include Garnier-Robson alpha-regions, beta-regions, turn-regions and coil-regions, Chou-Fasman alpha-regions, beta-regions and turn-regions, Kyte-Doolittle hydrophilic regions and hydrophilic regions, Eisenberg alpha and beta amphipathic regions, Karplus-Schulz flexible regions, Emini surface-forming regions and Jameson-Wolf high antigenic index regions.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The polypeptides of the present invention include the polypeptide of SBQ ID NO:2 (in particular the mature polypeptide) as well as polypeptides which have at least 80% similarity (preferably at least 80% identity) to the polypeptide of SEQ ID NO:2 and more preferably at least 90% similarity (more preferably at least 90% identity) to the polypeptide of SEQ ID NO:2 and still more preferably at least 95% similarity (still more preferably at least 95% identity) to the polypeptide of SBQ ID NO:2 and also include portions of such polypeptides with such portion of the polypeptide generally containing at least 30 amino acids and more preferably at least 50 amino acids.

As known in the art "similarity" between two polypeptides is determined by comparing the amino acid sequence and its conserved amino acid substitutes of one polypeptide to the sequence of a second polypeptide.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention.

The present invention also relates to vectors according to claim 5 which include polynucleotides of the present invention, host cells which are genetically engineered with vectors of the invention and the production of polypeptides of the invention by recombinant techniques.

Host cells according to claim 7 are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Spodoptera Sf9; animal cells such as CHO, COS or Bowes melanoma; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQB70, pQE60, pQE-9 (Qiagen), pBS, pD10, phagescript, psiX174, pBluescript SK, pBSKS, pNH8A, pNH16a, pNH18A, pNH46A (Stratagene); pTRC99a, pKK223-3, pKK233-3, pDR540, pRIT5 (Pharmacia); Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E . coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptide can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

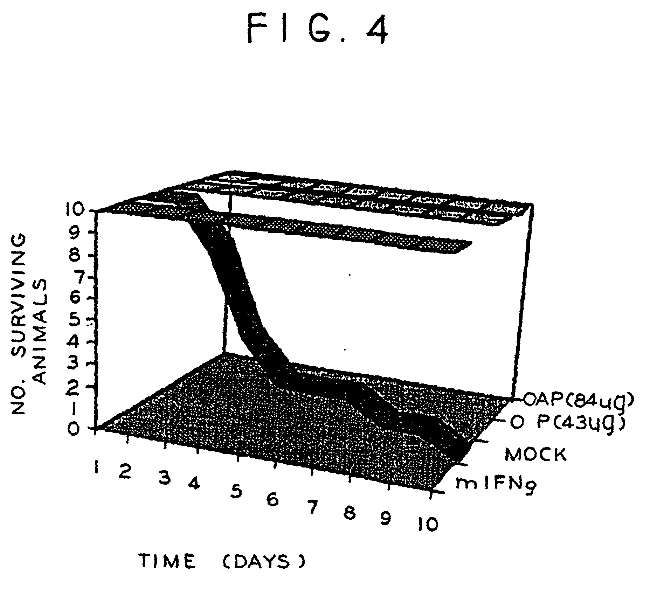

OAP may be employed as an antiviral agent. More particularly, OAP may be employed to treat necrotizing pancreatitis and parotitis caused by several members of the picornavirus family. As seen in Example 4 and Figure 4, OAP has an antiviral effect.

The polynucleotides and polypeptides of the present invention may also be employed as research reagents and materials for discovery of treatments and diagnostics to human disease.

Another potential antagonist according to claim 13 is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of OAP. The antisense RNA oligonucleotide hybridizes to the mRNA *in vivo* and blocks translation of the mRNA molecule into OAP polypeptide (Antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of OAP.

The antagonists may be employed in a composition with a pharmaceutically acceptable carrier according to claim 14, e.g., as hereinafter described.

The polypeptides of the present invention, and agonists and antagonists, may be employed in combination with a suitable pharmaceutical carrier. Such compositions comprise a therapeutically effective amount of the polypeptide, agonists or antagonist, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container (s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention, or agonists or antagonists, may be employed in conjunction with other therapeutic compounds.

The pharmaceutical compositions may be administered in a convenient manner such as by the oral, topical, parenterally, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The OAP polypeptides and agonists and antagonists which are polypeptides may also be employed according to claims 15-18 by expression of such polypeptides *in vivo,* which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art and are apparent from the teachings herein. For example, cells may be engineered by the use of a retroviral plasmid vector containing RNA encoding a polypeptide of the present invention.

Similarly, cells may be engineered *in vivo* for expression of a polypeptide *in vivo* by, for example, procedures known in the art. For example, a packaging cell is transduced with a retroviral plasmid vector containing RNA encoding a polypeptide of the present invention such that the packaging cell now produces infectious viral particles containing the gene of interest. These producer cells may be administered to a patient for engineering cells *in vivo* and expression of the polypeptide *in vivo.* These and other methods for administering a polypeptide of the present invention by such method should be apparent to those skilled in the art from the teachings of the present invention.

Retroviruses from which the retroviral plasmid vectors hereinabove mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

The vector includes one or more promoters. Suitable promoters which may be employed include, but are not limited to, the retroviral LTR; the SV40 promoter; and the human cytomegalovirus (GMV) promoter described in Miller, et al., Biotechniques, Vol. 7, No. 9, 980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The nucleic acid sequence encoding the polypeptide of the present invention is under the control of a suitable promoter. Suitable promoters which may be employed include, but are not limited to, adenoviral promoters, such as the adenoviral major late promoter; or hetorologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs hereinabove described); the β-actin promoter; and human growth hormone promoters. The promoter also may be the native promoter which controls the gene encoding the polypeptide.

The retroviral plasmid vector is employed to transduce packaging cell lines to form producer cell lines. Examples of packaging cells which may be transfected include, but are not limited to, the PE501, PA317, ψ-2, ψ-AM, PA12, T19-14X, VT-19-17-H2, ψCRE, ψCRIP, GP+E-86, GP+envAm12, and DAN cell lines as described in Miller, Human Gene Therapy, Vol. 1, pgs. 5-14 (1990), which is incorporated herein by reference in its entirety. The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles which include the nucleic acid sequence (s) encoding the polypeptides. Such retroviral vector particles then may be employed, to transduce eukaryotic cells, either *in vitro* or *in vivo.* The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

This invention is also related to the use of the gene of the present invention as a diagnostic. Detection of a mutated form of the gene will allow a diagnosis of a disease or a susceptibility to a disease which results from underexpression of OAP.

Individuals carrying mutations in the gene of the present invention may be detected at the DNA level by a variety of techniques. Nucleic acids for diagnosis may be obtained from a patient's cells, including but not limited to blood, urine, saliva, tissue biopsy and autopsy material. The genomic DNA may be used directly for detection or may be amplified enzymatically by using PCR (Saiki *et al.,* Nature, 324:163-166 (1986)) prior to analysis. RNA or cDNA may also be used for the same purpose. As an example, PCR primers complementary to the nucleic acid encoding OAP can be used to identify and analyze mutations. For example, deletions and insertions can be detected by a change in size of the amplified product in comparison to the normal genotype. Point mutations can be identified by hybridizing amplified DNA to radiolabeled RNA or alternatively, radiolabeled antisense DNA sequences. Perfectly matched sequences can be distinguished from mismatched duplexes by RNase A digestion or by differences in melting temperatures.

Sequence differences between the reference gene and genes having mutations may be revealed by the direct DNA sequencing method. In addition, cloned DNA segments may be employed as probes to detect specific DNA segments. The sensitivity of this method is greatly enhanced when combined with PCR. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al*., Science, 230:1242 (1985)).

Sequence changes at specific locations may also be revealed by nuclease protection assays, such as RNase and S1 protection or the chemical cleavage method (e.g., Cotton *et al*., PNAS, USA, 85:4397-4401 (1985)).

Thus, the detection of a specific DNA sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA.

In addition to more conventional gel-electrophoresis and DNA sequencing, mutations can also be detected by *in situ* analysis.

The present invention also relates to a diagnostic process according to claim 18 for detecting altered levels of the polypeptide of the present invention in various tissues since an over-expression of the proteins compared to normal control tissue samples can detect the presence of bone disorders, for example, osteoporosis. Assays used to detect levels of the polypeptide of the present invention in a sample derived from a host are well-known to those of skill in the art and include radioimmunoassays, competitive-binding assays, Western Blot analysis and preferably an ELISA assay. An ELISA assay initially comprises preparing an antibody specific to the OAP antigen, preferably a monoclonal antibody. In addition a reporter antibody is prepared against the monoclonal antibody. To the reporter antibody is attached a detectable reagent such as radioactivity, fluorescence or in this example a horseradish peroxidase enzyme. A sample is now removed from a host and incubated on a solid support, e.g. a polystyrene dish, that binds the proteins in the sample. Any free protein binding sites on the dish are then covered by incubating with a non-specific protein such as bovine serum albumin. Next, the monoclonal antibody is incubated in the dish during which time the monoclonal antibodies attached to any of the polypeptide of the present invention attached to the polystyrene dish. All unbound monoclonal antibody is washed out with buffer. The reporter antibody linked to horseradish peroxidase is now placed in the dish resulting in binding of the reporter antibody to any monoclonal antibody bound to the polypeptide of the present invention. Unattached reporter antibody is then washed out. Peroxidase substrates are then added to the dish and the amount of color developed in a given time period is a measurement of the amount of the polypeptide of the present invention present in a given volume of patient sample when compared against a standard curve.

A competition assay may be employed wherein antibodies specific to the polypeptide of the present invention are attached to a solid support and labeled OAP and a sample derived from the host are passed over the solid support and the amount of label detected attached to the solid support can be correlated to a quantity of the polypeptide of the present invention in the sample.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA having at least 50 or 60 bases. For a review of this technique, see Verma et al., Human Chromosomes: a Manual of Basic Techniques, Pergamon Press, New York (1988).

The OAP gene of the present invention has been mapped to human chromosome 1q21. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies according to claim 11. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Cloning and expression of OAP using the baculovirus expression system

The DNA sequence encoding the full length OAP protein, ATCC # 97302, was amplified using PCR oligonucleotide primers corresponding to the 5' and 3' sequences of the gene:

The 5' primer has the sequence 5' CGGGATCCGCCATCATGGGG ACCACAGCCAG 3' (SEQ ID NO:3) and contains a BamHI restriction enzyme site (in bold) followed by nucleotides resembling an efficient signal for the initiation of translation in eukaryotic cells (Kozak, M., J. Mol. Biol., 196:947-950 (1987) just in front of the the initiation codon for translation "ATG" (underlined).

The 3' primer has the sequence 5' GCTCTAGATCCAAGAGGTGTTT AGTG 3' (SEQ ID NO:4) and contains the cleavage site for the restriction endonuclease XbaI and 18 nucleotides complementary to the 3' non-translated sequence. The amplified sequences were isolated from a 1% agarose gel using a commercially available kit ("Geneclean," BIO 101 Inc., La Jolla, Ca.). The fragment was then digested with the endonucleases BamHI and XbaI and then purified again on a 1% agarose gel. This fragment is designated F2.

The vector pA2 (modification of pVL941 vector, discussed below) is used for the expression of the OAP protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhedrosis virus (AcMNPV) followed by the recognition sites for the restriction endonucleases BamHI and XbaI. The polyadenylation site of the simian virus (SV)40 is used for efficient polyadenylation. For an easy selection of recombinant virus the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of co-transfected wild-type viral DNA. Many other baculovirus vectors could be used in place of pA2 such as pAc373, pVL941 and pAcIM1 (Luckow, V.A. and Summers, M.D., Virology, 170:31-39).

The plasmid was digested with the restriction enzymes BamHI and XbaI and then dephosphorylated using calf intestinal phosphatase by procedures known in the art. The DNA was then isolated from a 1% agarose gel using the commercially available kit ("Geneclean" BIO 101 Inc., La Jolla, Ca.). This vector DNA is designated V2.

Fragment F2 and the dephosphorylated plasmid V2 were ligated with T4 DNA ligase. E.coli HB101 cells were then transformed and bacteria identified that contained the plasmid (pBacOAP) with the OAP gene using the enzymes BamHI and XbaI. The sequence of the cloned fragment was confirmed by DNA sequencing.

5 µg of the plasmid pBacOAP was co-transfected with 1.0 µg of a commercially available linearized baculovirus ("BaculoGold™ baculovirus DNA", Pharmingen, San Diego, CA.) using the lipofection method (Pelgner et al. Proc. Natl. Acad. Sci. USA, 84:7413-7417 (1987)).

1µg of BaculoGold™ virus DNA and 5 µg of the plasmid pBacOAP were mixed in a sterile well of a microtiter plate containing 50 µl of serum free Grace's medium (Life Technologies Inc., Gaithersburg, MD). Afterwards 10 µl Lipofectin plus 90 µl Grace's medium were added, mixed and incubated for 15 minutes at room temperature. Then the transfection mixture was added drop-wise to the Sf9 insect cells (ATCC CRL 1711) seeded in a 35 mm tissue culture plate with 1 ml Grace's medium without serum. The plate was rocked back and forth to mix the newly added solution. The plate was then incubated for 5 hours at 27°C. After 5 hours the transfection solution was removed from the plate and 1 ml of Grace's insect medium supplemented with 10% fetal calf serum was added. The plate was put back into an incubator and cultivation continued at 27°C for four days.

After four days the supernatant was collected and a plaque assay performed similar as described by Summers and Smith (supra). As a modification an agarose gel with "Blue Gal" (Life Technologies Inc., Gaithersburg) was used which allows an easy isolation of blue stained plaques. (A detailed description of a "plaque assay" can also be found in the user's guide for insect cell culture and baculovirology distributed by Life Technologies Inc., Gaithersburg, page 9-10).

Pour days after the serial dilution, the virus was added to the cells and blue stained plaques were picked with the tip of an Eppendorf pipette. The agar containing the recombinant viruses was then resuspended in an Eppendorf tube containing 200 µl of Grace's medium. The agar was removed by a brief centrifugation and the supernatant containing the recombinant baculovirus was used to infect Sf9 cells seeded in 35 mm dishes. Four days later the supernatants of these culture dishes were harvested and then stored at 4°C.

Sf9 cells were grown in Grace's medium supplemented with 10% heat-inactivated FBS. The cells were infected with the recombinant baculovirus V-OAP at a multiplicity of infection (MOI) of 2. Six hours later the medium was removed and replaced with SF900 II medium minus methionine and cysteine (Life Technologies Inc., Gaithersburg). 42 hours later 5 µCi of ³⁵S-methionine and 5 µCi ³⁵S cysteine (Amersham) were added. The cells were further incubated for 16 hours before they were harvested by centrifugation and the labelled proteins visualized by SDS-PAGE and autoradiography.

The supernatant (1000 ml) containing baculovirus expressed OAP was applied without dilution to an HS-50 column (1.0 x 10cm, perseptive Biosystems) equilibrated with 0.02M Bis-Tris, pH 6.0, containing 10% glycerol and 0.02 M NaCl (Solvent A) at a flow rate of 8 ml/min. Proteins were then eluted using a gradient from 10% Solvent B (Solvent A containing 2 M NaCl) to 30% B. The pooled peak contained 11 mg of OAP having a purity of greater than 80%.

### Example 2

### Expression of Recombinant OAP in COS cells

The expression of plasmid, OAP HA is derived from a vector pcDNAI/Amp (Invitrogen) containing: 1) SV40 origin of replication, 2) ampicillin resistance gene, 3) E.coli replication origin, 4) CMV promoter followed by a polylinker region, an SV40 intron and polyadenylation site. A DNA fragment encoding the entire OAP precursor and a HA tag fused in frame to its 3' end was cloned into the polylinker region of the vector, therefore, the recombinant protein expression is directed under the CMV promoter. The HA tag corresponds to an epitope derived f rom the influenza hemagglutinin protein as previously described (I. Wilson, H. Niman, R. Heighten, A Cherenson, M. Connolly, and R. Lerner, 1984, Cell 37:767, (1984)). The infusion of HA tag to the target protein allows easy detection of the recombinant protein with an antibody that recognizes the HA epitope.

The plasmid construction strategy is described as follows:

The DNA sequence encoding OAP, ATCC # 97302, was constructed by PCR using two primers: the 5' primer 5' GCGCGGATCCACCATGGGGACCACAGCCAGA 3' (SEQ ID NO:5) contains a BamHI site followed by 18 nucleotides of OAP coding sequence starting from the initiation codon; the 3' sequence 5 GCGCTCTAGATCAAGCGTAGTCTGGGACGTCGTATGGGTATTCTTCCTTGGGCTC3' (SEQ ID NO:6) contains complementary sequences to an XbaI site, translation stop codon, HA tag and the last 15 nucleotides of the OAP coding sequence (not including the stop codon). Therefore, the PCR product contains a BamHI site, OAP coding sequence followed by HA tag fused in frame, a translation termination stop codon next to the HA tag, and an XbaI site. The PCR amplified DNA fragment and the vector, pcDNAI/Amp, were digested with BamHI and XbaI restriction enzyme and ligated. The ligation mixture was transformed into E. coli strain SURE (available from Stratagene Cloning Systems, 11099 North Torrey Pines Road, La Jolla, CA 92037) the transformed culture was plated on ampicillin media plates and resistant colonies were selected. Plasmid DNA was isolated from transformants and examined by restriction analysis for the presence of the correct fragment. For expression of the recombinant OAP, COS cells were transfected with the expression vector by DEAE-DEXTRAN method (J. Sambrook, E. Fritsch, T. Maniatis, Molecular Cloning: A Laboratory Manual, Cold Spring Laboratory Press, (1989)). The expression of the OAP HA protein was detected by radiolabelling and immunoprecipitation method (E. Harlow, D. Lane, Antibodies: A Laboratory Manual, Cold Spring Harbor Laboratory Press, (1988)). Cells were labelled for 8 hours with ³⁵S-cysteine two days post transfection. Culture media was then collected and cells were lysed with detergent (RIPA buffer (150 mM NaCl, 1% NP-40, 0.1% SDS, 1% NP-40, 0.5% DOC, 50mM Tris, pH 7.5) (Wilson, I. et al., Id. 37:767 (1984)). Both cell lysate and culture media were precipitated with an HA specific monoclonal antibody. Proteins precipitated were analyzed on 15% SDS-PAGE gels.

### Example 3

### Expression via Gene Therapy

Fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin, is added. This is then incubated at 37°C for approximately one week. At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al, DNA, 7:219-25 (1988) flanked by the long terminal repeats of the Moloney murine sarcoma virus, is digested with EcoRI and HindIII and subsequently treated with calf intestinal phosphatase. The linear vector is fractionated on agarose gel and purified, using glass beads. -

The cDNA encoding a polypeptide of the present invention is amplified using PCR primers which correspond to the 5' and 3' end sequences respectively. The 5' primer containing an EcoRI site and the 3' primer further includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is used to transform bacteria HB101, which are then plated onto agar-containing kanamycin for the purpose of confirming that the vector had the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells are transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his.

The engineered fibroblasts are then injected into the host, either alone or after having been grown to confluence on cytodex 3 microcarrier beads. The fibroblasts now produce the protein product.

### Example 4

### Anti-Viral Effect of OAP

Several members of the picornavirus family cause acute necrotizing pancreatitis and parotitis in mice (Barger, M.T. and Craighead, J.E., 1991; Babu, P.G. , Huber, S.A. Craighead, J.E., 1986; Blay, R., Simpson K., Leslie, K., and Huber, S. A. 1989; Campbell, I.L. et al,, 1985). The actual pathogenesis of virus-induced changes is unknown; although, it is generally agreed that immunopathogenic mechanisms are involved (Barger, M.T. and Craighead, J.E., 1991). A protective effect against subsequent infection with EMCV has been demonstrated in mice (Burger, M. T. and Craighead, J.E. 1991). The present experiment demonstrates the anti-viral effect of the OAP treatment over the course of EMCV infection in the mouse using similar methods.

### Mice

Male, 8 to 10 week-old A/J mice purchased from the Jackson Laboratory (Bar Harbor ME) were maintained in caged groups of ten animals at room temperature with alternate 12 hour periods of light and dark. Animals had access to food and water ad libitum. There were no special dietary arrangements.,

### Virus

The E variant strain of EMCV was obtained froth the ATCC (Rockville, Maryland) and propagated in L929 cells via direct infection. Plaque supernatant viral titers were assayed on L929 cells, and TCID5O values were calculated as described above.

### Infection protocol

Prior to performing protection experiments the dose of EMCV required to kill 50% of the animals was determined ("LD50"). Typically, EMCV infection kills animals after seven to ten days. 10 infectious units of EMCV used in these experiments was effective to kill 50% of the animals by day seven. To determine the optimum doses of murine IFN-gamma and alpha:beta interferon to afford protection from EMCV-induced pathological effects, groups of ten animals kept in the same cage were pretreated, per animal, with 1000 units of murine IFN-gamma or alpha:beta interferon mixture, followed by intraperitoneal (i.p.) inoculation with 10 infectious units of EMCV in Hank's balanced salt solution (HBSS). In parallel, duplicate numbers of animals were pretreated in the same way with saline or approximately 500 IFN-equivalent units of OAP, followed by EMCV inoculation 24 hours later. Animals were observed 10 days thereafter for signs of EMCV-related pathology.

### Analysis

Figure 4 depicts graphically the effects of EMCV challenge on each group of mice. This graph of the number of surviving mice in each group ondays 1-10 after EMCV chanllenge show that OAP protected mice against EMCV at least as well as murine IFN. All mice treated with either dose of OAP, and those treated with mIFN survived past day 10, whereas by then all mock-injected mice had died.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as depicted in SEQ ID NO: 2;
(b) polynucleotides encoding a polypeptide comprising amino acids 20 to 540 of SEQ ID NO:2;
(c) polynucleotides encoding a polypeptide comprising amino acids 1 to 540 of SEQ ID NO: 2;
(d) polynucleotides having the coding sequence as depicted in SEQ ID NO: 1 encoding at least the mature form of the polypeptide;
(e) polynucleotides comprising nucleotides 82 to 1701 depicted in SEQ ID NO: 1;
(f) polynucleotides comprising nucleotides 139 to 1701 depicted in SEQ ID NO: 1;
(g) polynucleotides encoding the polypeptide having the amino acid sequence of at least the mature form of the polypeptide encoded by the cDNA contained in ATCC 97302;
(h) polynucleotides having the coding sequence of the cDNA contained in ATCC 97302 encoding at least the mature form of the polypeptide;
(i) polynucleotides having the coding sequence of the cDNA contained in ATCC 97302;
(j) polynucleotides encoding a first polypeptide which is at least 80% identical to a second polypeptide encoded by a polynucleotide of any one of (a) to (h), wherein said first polypeptide has antiviral activity to EMCV; and
(k) polynucleotides the complementary strand of which hybridizes with and is at least 85% identical to a polynucleotide as defined in any one of (a) to (h), wherein said polynucleotides encode a polypeptide having antiviral activity to EMCV;
or the complementary strand of such a polynucleotide.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell containing the vector of claim 5 or 6.

8. A process for producing a polypeptide encoded by the polynucleotide of any one of claims 1 to 4 comprising: culturing the host cell of claim 7 and recovering said polypeptide encoded by said polynucleotide from the culture.

9. A process for producing cells capable of expressing a polypeptide encoded by the polynucleotide of any one of claims 1 to 4 comprising genetically engineering cells with the vector of claim 5 or 6.

10. A polypeptide having the amino acid sequence encoded by a polynucleotide of any one of claims 1 to 4 or obtainable by the process of claim 8.

11. An antibody specifically binding to the polypeptide of claim 10 which has antiviral activity to EMCV.

12. A nucleic acid molecule which specifically hybridizes to a polynucleotide of any one of claims 1 to 4 and which is at least 90% identical to said polynucleotide.

13. An antagonist/inhibitor of the receptor for the polypeptide of claim 10, wherein the antagonist/inhibitor is an antisense DNA or RNA specifically hybridizing to and being at least 90% identical to a polynucleotide of any one of claims 1 to 4 or an antibody specific for the polypeptide of claim 10.

14. A pharmaceutical composition comprising the polynucleotide of any one of claims 1 to 4, the polypeptide of claim 10 or a DNA encoding and capable of expressing said polypeptide in vivo or the antagonist/inhibitor of claim 13, and optionally a pharmaceutically acceptable carrier.

15. A diagnostic composition comprising the polynucleotide of any one of claims 1 to 4, the nucleic acid molecule of claim 12 or the antibody of claim 11.

16. Use of the polypeptide of claim 10, the polynucleotide of any one of claims 1 to 4 or the vector of claim 5 or 6 for the preparation of a pharmaceutical composition for treating a disease caused by infection with a virus.

17. A process for diagnosing a disease or a susceptibility to a disease related to the polypeptide of claim 10 comprising determining ex vivo a mutation in a nucleic acid sequence encoding said polypeptide.

18. A diagnostic process comprising analyzing for the presence of the polypeptide of claim 10 in a sample derived from a host.

## Patentansprüche

1. Polynucleotid, ausgewählt aus der Gruppe bestehend aus
(a) Polynucleotiden, die zumindest die reife Form des Polypeptids codieren, das die abgeleitete, wie in SEQ ID NO: 2 dargestellte Aminosäuresequenz hat;
(b) Polynucleotiden, die ein Polypeptid codieren, das die Aminosäuren 20 bis 540 von SEQ ID NO: 2 umfasst;
(c) Polynucleotiden, die ein Polypeptid codieren, das die Aminosäuren 1 bis 540 von SEQ ID NO: 2 umfasst;
(d) Polynucleotiden, die die in SEQ ID NO: 1 dargestellte codierende Sequenz haben, die zumindest die reife Form des Polypeptids codiert;
(e) Polynucleotiden, umfassend die in SEQ ID NO: 1 dargestellten Nucleotide 82 bis 1701;
(f) Polynucleotiden, umfassend die in SEQ ID NO: 1 dargestellten Nucleotide 139 bis 1701;
(g) Polynucleotiden, die das Polypeptid codieren, das die Aminosäuresequenz von zumindest der reife Form des Polypeptids hat, das durch die in ATCC 97302 enthaltene cDNA codiert wird;
(h) Polynucleotiden, die die codierende Sequenz der in ATCC 97302 enthaltenen cDNA haben, die zumindest die reife Form des Polypeptids codiert;
(i) Polynucleotiden, die die codierende Sequenz der in ATCC 97302 enthaltenen cDNA haben;
(j) Polynucleotiden, die ein erstes Polypeptid codieren, das zumindest zu 80% mit einem zweiten Polypeptid identisch ist, das von einem Polynucleotid nach einem der Punkte (a) bis (h) codiert wird, wobei das erste Polypeptid antivirale Aktivität gegen EMCV hat; und
(k) Polynucleotiden, deren komplementärer Strang mit einem in einem der Punkte (a) bis (h) definierten Polynucleotid hybridisiert und zumindest zu 85% damit identisch ist, wobei die Polynucleotide ein Polypeptid codieren, das antivirale Aktivität gegen EMCV hat;
oder der komplementäre Strang eines solchen Polynucleotids.

2. Polynucleotid nach Anspruch 1, das DNA ist.

3. DNA nach Anspruch 2, die genomische DNA ist.

4. Polynucleotid nach Anspruch 1, das RNA ist.

5. Vektor, der das Polynucleotid nach einem der Ansprüche 1 bis 4 enthält.

6. Vektor nach Anspruch 5, in dem das Polynucleotid funktionell mit Expressionskontrollsequenzen verbunden ist, die eine Expression in prokaryontischen oder eukaryontischen Wirtszellen erlauben.

7. Wirtszelle, die den Vektor nach Anspruch 5 oder 6 enthält.

8. Verfahren zur Herstellung eines Polypeptids, das durch das Polynucleotid nach einem der Ansprüche 1 bis 4 codiert wird, umfassend: Züchten der Wirtszelle nach Anspruch 7 und Gewinnen des durch das Polynucleotid codierten Polypeptids aus der Kultur.

9. Verfahren zur Herstellung von Zellen, die in der Lage sind, ein Polypeptid, das durch das Polynucleotid nach einem der Ansprüche 1 bis 4 codiert wird, zu exprimieren, umfassend das genetische Verändern von Zellen mit dem Vektor nach Anspruch 5 oder 6.

10. Polypeptid, das die Aminosäuresequenz hat, die von einem Polynucleotid nach einem der Ansprüche 1 bis 4 codiert wird, oder das durch das Verfahren nach Anspruch 8 erhältlich ist.

11. Antikörper, der spezifisch an das Polypeptid nach Anspruch 10, das antivirale Aktivität gegen EMCV hat, bindet.

12. Nucleinsäuremolekül, das spezifisch an ein Polynucleotid nach einem der Ansprüche 1 bis 4 hybridisiert und das zumindest zu 90% mit dem Polynucleotid identisch ist.

13. Antagonist/Inhibitor des Rezeptors für das Polypeptid nach Anspruch 10, wobei der Antagonist/Inhibitor eine Antisense-DNA oder -RNA ist, die spezifisch mit einem Polynucteotid nach einem der Ansprüche 1 bis 4 hybridisiert und zumindest zu 90% damit identisch ist, oder der ein Antikörper ist, der spezifisch für das Polypeptid nach Anspruch 10 ist.

14. Arzneimittel, umfassend das Polynucleotid nach einem der Ansprüche 1 bis 4, das Polypeptid nach Anspruch 10 oder eine DNA, die das Polypeptid codiert und in der Lage ist, es in vivo zu exprimieren, oder den Antagonisten/Inhibitor nach Anspruch 13, und gegebenenfalls einen pharmazeutisch verträglichen Träger.

15. Diagnostische Zusammensetzung, umfassend das Polynucleotid nach einem der Ansprüche 1 bis 4, das Nucleinsäuremolekül nach Anspruch 12 oder den Antikörper nach Anspruch 11.

16. Verwendung des Polypeptids nach Anspruch 10, des Polynucleotids nach einem der Ansprüche 1 bis 4 oder des Vektors nach Anspruch 5 oder 6 für die Herstellung eines Arzneimittels zur Behandlung einer durch Infektion mit einem Virus verursachten Krankheit.

17. Verfahren zur Diagnose einer Krankheit oder einer Anfälligkeit für eine Krankheit, die mit dem Polypeptid nach Anspruch 10 in Bezug steht, umfassend die ex vivo-Bestimmung einer Mutation in einer Nucleinsäuresequenz, die das Polypeptid codiert.

18. Diagnostisches Verfahren, umfassend das Analysieren auf die Anwesenheit des Polypeptids nach Anspruch 10 in einer von einem Wirt stammenden Probe.

## Revendications

1. Polynucléotide choisi dans le groupe constitué par
(a) des polynucléotides codant pour au moins la forme mature du polypeptide présentant la séquence d'acides aminés déduite décrite dans SEQ ID N° : 2 ;
(b) des polynucléotides codant pour un polypeptide comprenant les acides aminés 20 à 540 de SEQ ID N° : 2 ;
(c) des polynucléotides codant pour un polypeptide comprenant les acides aminés 1 à 540 de SEQ ID N° : 2 ;
(d) des polynucléotides présentant la séquence codante telle que décrite dans SEQ ID N° : 1 codant pour au moins la forme mature du polypeptide ;
(e) des polynucléotides comprenant les nucléotides 82 à 1701 décrits dans SEQ ID N° : 1 ;
(f) des polynucléotides comprenant les nucléotides 139 à 1701 décrits dans SEQ ID N° : 1 ;
(g) des polynucléotides codant pour le polypeptide présentant la séquence d'acides aminés d'au moins la forme mature du polypeptide codé par l'ADNc contenu dans l'ATCC 97302 ;
(h) des polynucléotides présentant la séquence codante de l'ADNc contenue dans l'ATCC 97302 codant pour au moins la forme mature du polypeptide ;
(i) des polynucléotides présentant la séquence codante de l'ADNc contenue dans l'ATCC 97302 ;
(j) des polynucléotides codant pour un premier polypeptide qui est au moins à 80 % identique à un deuxième polypeptide codé par un polynucléotide de l'un quelconque des polynucléotides (a) à (h), dans lesquels ledit premier polypeptide présente une activité antivirale contre l'EMCV ; et
(k) des polynucléotides dont le brin complémentaire s'hybride avec, et est au moins identique à 85 % à, un polynucléotide tel que défini dans l'un quelconque des polynucléotides (a) à (h), dans lesquels lesdits polynucléotides codent pour un polypeptide présentant une activité antivirale contre l'EMCV ;
ou le brin complémentaire de ce polynucléotide.

2. Polynucléotide selon la revendication 1 qui est un ADN.

3. ADN de la revendication 2 qui est un ADN génomique.

4. Polynucléotide de la revendication 1 qui est un ARN.

5. Vecteur contenant le polynucléotide de l'une quelconque des revendications 1 à 4.

6. Vecteur selon la revendication 5 dans lequel le polynucléotide est lié de manière fonctionnelle à des séquences de contrôle de l'expression permettant l'expression dans des cellules hôtes procaryotes ou eucaryotes.

7. Cellule hôte contenant le vecteur de la revendication 5 ou 6.

8. Procédé de production d'un polypeptide codé par le polynucléotide de l'une quelconque des revendications 1 à 4 comprenant la culture de la cellule hôte de la revendication 7 et la récupération dudit polypeptide codé par ledit polynucléotide à partir de la culture.

9. Procédé de production de cellules pouvant exprimer un polypeptide codé par le polynucléotide de l'une quelconque des revendications 1 à 4 comprenant la manipulation génétique des cellules avec le vecteur de la revendication 5 ou 6.

10. Polypeptide présentant la séquence d'acides aminés codée par un polynucléotide selon l'une quelconque des revendications 1 à 4 ou pouvant être obtenu par le procédé de la revendication 8.

11. Anticorps se liant spécifiquement au polypeptide de la revendication 10 qui a une activité antivirale contre l'EMCV.

12. Molécule d'acide nucléique qui s'hybride spécifiquement à un polynucléotide de l'une quelconque des revendications 1 à 4 qui est au moins à 90 % identique au dit polynucléotide.

13. Antagoniste/inhibiteur du récepteur du polypeptide de la revendication 10, dans lequel l'antagoniste/inhibiteur est un ADN ou un ARN anti-sens s'hybridant spécifiquement à, et étant identique à au moins 90 % à, un polynucléotide de l'une quelconque des revendications 1 à 4, ou un anticorps spécifique du polypeptide de la revendication 10.

14. Composition pharmaceutique comprenant le polynucléotide de l'une quelconque des revendications 1 à 4, le polypeptide de la revendication 10 ou un ADN codant pour et capable d'exprimer ledit polypeptide *in vivo* ou l'antagoniste/inhibiteur de la revendication 13 et éventuellement, un véhicule pharmaceutiquement acceptable.

15. Composition diagnostique comprenant le polynucléotide de l'une quelconque des revendications 1 à 4, la molécule d'acide nucléique de la revendication 12 ou l'anticorps de la revendication 11.

16. Utilisation du polypeptide de la revendication 10, du polynucléotide de l'une quelconque des revendications 1 à 4 ou du vecteur de la revendication 5 ou 6 pour la préparation d'une composition pharmaceutique pour traiter une maladie provoquée par une infection par un virus.

17. Procédé de diagnostic d'une maladie ou d'une prédisposition à une maladie liée au polypeptide de la revendication 10, comprenant la détermination *ex vivo* d'une mutation dans une séquence d'acide nucléique codant pour ledit polypeptide.

18. Procédé diagnostique comprenant l'analyse de la présence du polypeptide de la revendication 10 dans un échantillon dérivé d'un hôte.


### SEQUENCE LISTING

(1) GENERAL INFORMATION:
   (i) APPLICANT: NI, Jian .
      DILLON, Patrick J.
      GENTZ, Reiner L.
   (ii) TITLE OF INVENTION: Osteo Antiviral Protein
   (iii) NUMBER OF SEQUENCES: 7
   (iv) CORRESPONDENCE ADDRESS:
      (A) ADDRESSEE: Carella, Byrne, Bain, Gilfillan, Cecchi, Stewart & Olstein
      (B) STREET: 6 Becker Farm Road
      (C) CITY: Roseland
      (D) STATE: NJ
      (E) COUNTRY: USA
      (F) ZIP: 07068-1739
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Ferraro, Gregory D
      (B) REGISTRATION NUMBER: 36,134
      (C) REFERENCE/DOCKET NUMBER: 325800-486
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS:
      (A) LENGTH: 1816 base pairs
      (B) TYPE: nucleic acid
      (C) STRANDEDNESS: single
      (D) TOPOLOGY: linear.
   (ii) MOLECULE TYPE: DNA (genomic)
   (ix) FEATURE:
      (A) NAME/KEY: CDS
      (B) LOCATION: 82..1701
   (xi) SEQUENCE DESCRIPTION: SEQ ID NO:1:
   AAGCTTCAGT GGCCGGCCCT TCACATCCAG ACTTGCCTGA GAGGACCCAC CTCTGAGTGT       60
   CCAGTGGTCA GTTGCCCCAG G ATG GGG ACC ACA GCC AGA GCA GCC TTG GTC        111
                           Met Gly Thr Thr Ala Arg Ala Ala Leu Val
                            1               5                  10
   TTG ACC TAT TTG GCT GTT GCT TCT GCT GCC TCT GAG GGA GGC TTC ACG     159
   Leu Thr Tyr Leu Ala Val Ala Ser Ala Ala Ser Glu Gly Gly Phe Thr
                   15                  20                  25
   GCT ACA GGA CAG AGG CAG CTG AGG CCA GAG CAC TTT CAA GAA GTT GGC     207
   Ala Thr Gly Gln Arg Gln Leu Arg Pro Glu His Phe Gln Glu Val Gly
               30                  35                  40
   TAC GCA GCT CCC CCC TCC CCA CCC CTA TCC CGA AGC CTC CCC ATG GAT     255
   Tyr Ala Ala Pro Pro Ser Pro Pro Leu Ser Arg Ser Leu Pro Met Asp
           45                  50                  55
   CAC CCT GAC TCC TCT CAG CAT GGC CCT CCC TTT GAG GGA CAG AGT CAA     303
   His Pro Asp Ser Ser Gln His Gly Pro Pro Phe Glu Gly Gln Ser Gln
       60                  65                  70
   GTG CAG CCC CTC CCT CTC AGG AGG CCA CCC CTC TTC CAA CAG GAA AAG     351
   Val Gln Pro Leu Pro Leu Arg Arg Pro Pro Leu Phe Gln Gln Glu Lys
   75                  80                  85                  90
   CTG CTA CCT GCC CAA CTC CCT GCT GAA AAG GAA GTG GGT CCC CCT CTC     399
   Leu Leu Pro Ala Gln Leu Pro Ala Glu Lys Glu Val Gly Pro Pro Leu
                   95                  100                 105
   CCT CAG GAA GCT GTC CCC CTC CAA AAA GAG CTG CCC TCT CTC CAG CAC     447
   Pro Gln Glu Ala Val Pro Leu Gln Lys Glu Leu Pro Ser Leu Gln His
               110                 115                 120
   CCC AAT GAA CAG AAG GAA GGA ACG CCA GCT CCA TTT GGG GAC CAG AGC     495
   Pro Asn Glu Gln Lys Glu Gly Thr Pro Ala Pro Phe Gly Asp Gln Ser
           125                 130                 135
   CAT CCA GAA CCT GAG TCC TGG AAT GCA GCC CAG CAC TGC CAA CAG GAC     543
   His Pro Glu Pro Glu Ser Trp Asn Ala Ala Gln His Cys Gln Gln Asp
       140                 145                 150
   CGG TCC CAA GGG GGC TGG GGC CAC CGG CTG GAT GGC TTC CCC CCT GGG     591
   Arg Ser Gln Gly Gly Trp Gly His Arg Leu Asp Gly Phe Pro Pro Gly
   155                 160                 165                 170
   CGG CCT TCT CCA GAC AAT CTG AAC CAA ATC TGC CTT CCT AAC CGT CAG     639
   Arg Pro Ser Pro Asp Asn Leu Asn Gln Ile Cys Leu Pro Asn Arg Gln
                   175                 180                 185
   CAT GTG GTA TAT GGT CCC TGG AAC CTA CCA CAG TCC AGC TAC TCC CAC     687
   His Val Val Tyr Gly Pro Trp Asn Leu Pro Gln Ser Ser Tyr Ser His
               190                 195                 200
   CTC ACT CGC CAG GGT GAG ACC CTC AAT TTC CTG GAG ATT GGA TAT TCC     735
   Leu Thr Arg Gln Gly Glu Thr Leu Asn Phe Leu Glu Ile Gly Tyr Ser
           205                 210                 215
   CGC TGC TGC CAC TGC CGC AGG CAC ACA AAC CGC CTA GAG TGT GCC AAA     783
   Arg Cys Cys His Cys Arg Arg His Thr Asn Arg Leu Glu Cys Ala Lys
       220                 225                 230
   CTT GTG TGG GAG GAA GCA ATG AGC CGA TTC TGT GAG GCC GAG TTC TCG     831
   Leu Val Trp Glu Glu Ala Met Ser Arg Phe Cys Glu Ala Glu Phe Ser
   235                 240                 245                 250
   GTC AAG ACC CGA CCC CAC TGG TGC TGC ACG CGG CAG GGG GAG GCT CGG     879
   Val Lys Thr Arg Pro His Trp Cys Cys Thr Arg Gln Gly Glu Ala Arg
                   255                 260                 265
   TTC TCC TGC TTC CAG GAG GAA GCT CCC CAG CCA CAC TAC CAG CTC CGG     927
   Phe Ser Cys Phe Gln Glu Glu Ala Pro Gln Pro His Tyr Gln Leu Arg
               270                 275                 280
   GCC TGC CCC AGC CAT CAG CCT GAT ATT TCC TCG GGT CTT GAG CTG CCT        975
   Ala Cys Pro Ser His Gln Pro Asp Ile Ser Ser Gly Leu Glu Leu Pro
           285                 290                 295
   TTC CCT CCT GGG GTG CCA CAT TGG ACA ATA TCA AGA ACA TCT TGC CAC       1023
   Phe Pro Pro Gly Val Pro His Trp Thr Ile Ser Arg Thr Ser Cys His
       300                 305                 310
   CTG AGG CGC TTC CGC TCT GTG CCA CGC AAC CTG CCA GCT ACT GAC CCC       1071
   Leu Arg Arg Phe Arg Ser Val Pro Arg Asn Leu Pro Ala Thr Asp Pro
   315                 320                 325                 330
   CTA CAA AGG GAG CTG CTG GCA CTG ATC CAG CTG GAG AGG GAG TTC CAG       1119
   Leu Gln Arg Glu Leu Leu Ala Leu Ile Gln Leu Glu Arg Glu Phe Gln
                   335                 340                 345
   CGC TGC TGC CGC CAG GGG AAC AAT CAC ACC TGT ACA TGG AAG GCC TGG       1167
   Arg Cys Cys Arg Gln Gly Asn Asn His Thr Cys Thr Trp Lys Ala Trp
               350                 355                 360
   GAG GAT ACC CTT GAC AAA TAC TGT GAC CGG GAG TAT GCT GTG AAG ACC       1215
   Glu Asp Thr Leu Asp Lys Tyr Cys Asp Arg Glu Tyr Ala Val Lys Thr
           365                 370                 375
   CAC CAC CAC TTG TGT TGG CGC CAC CCT CCC AGC CCT ACT CGG GAT GAG       1263
   His His His Leu Cys Trp Arg His Pro Pro Ser Pro Thr Arg Asp Glu
       380                 385                 390
   TGC TTT GGC CGT CGG GCT CCT TAC CCC AAC TAT GAC CGG GAC ATC TTG       1311
   Cys Phe Gly Arg Arg Ala Pro Tyr Pro Asn Tyr Asp Arg Asp Ile Leu
   395                 400                 405                 410
   ACC ATT GAC ATC GGT CGA GTC ACC CCC AAC CTC ATG GGC CAC CTC TGT       1359
   Thr Ile Asp Ile Gly Arg Val Thr Pro Asn Leu Met Gly His Leu Cys
                   415                 420                 425
   GGA AAC CAA AGA GTT CTC ACC AAG CAT AAA CAT ATT CCT GGG CTG ATC       1407
   Gly Asn Gln Arg Val Leu Thr Lys His Lys His Ile Pro Gly Leu Ile
               430                 435                 440
   CAC AAC ATG ACT GCC CGC TGC TGT GAC CTG CCA TTT CCA GAA CAG GCC       1455
   His Asn Met Thr Ala Arg Cys Cys Asp Leu Pro Phe Pro Glu Gln Ala
           445                 450                 455
   TGC TGT GCA GAG GAG GAG AAA TTA ACC TTC ATC AAT GAT CTG TGT GGT       1503
   Cys Cys Ala Glu Glu Glu Lys Leu Thr Phe Ile Asn Asp Leu Cys Gly
       460                 465                 470
   CCC CGA CGT AAC ATC TGG CGA GAC CCT GCC CTC TGC TGT TAC CTG AGT       1551
   Pro Arg Arg Asn Ile Trp Arg Asp Pro Ala Leu Cys Cys Tyr Leu Ser
   475                 480                 485                 490
   CCT GGG GAT GAA CAG GTC AAC TGC TTC AAC ATC AAT TAT CTG AGG AAC       1599
   Pro Gly Asp Glu Gln Val Asn Cys Phe Asn Ile Asn Tyr Leu Arg Asn
                   495                 500                 505
   GTG GCT CTA GTG TCT GGA GAC ACT GAG AAC GCC AAG GGC CAG GGG GAG       1647
   Val Ala Leu Val Ser Gly Asp Thr Glu Asn Ala Lys Gly Gln Gly Glu
               510                 515                 520
   CAG GGC TCA ACT GGA GGA ACA AAT ATC AGC TCC ACC TCT GAG CCC AAG       1695
   Gln Gly Ser Thr Gly Gly Thr Asn Ile Ser Ser Thr Ser Glu Pro Lys
           525                 530                 535
   GAA GAA TGAGTCACCC CAGAGCCCTA GAGGGTCAGA TGGGGGGAAC CCCACCCTGC        1751
   Glu Glu
       540
   CCCACCCATC TGAACACTCA TTACACTAAA CACCTCTTGG ATTTGGTGTC AAAAAAAAAA 1811
   AAAAA 1816
(2) INFORMATION FOR SEQ ID NO:2:
   (i) SEQUENCE CHARACTERISTICS:
      (A) LENGTH: 540 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gly Thr Thr Ala Arg Ala Ala Leu Val Leu Thr Tyr Leu Ala Val
    1               5                  10                  15
   Ala Ser Ala Ala Ser Glu Gly Gly Phe Thr Ala Thr Gly Gln Arg Gln
               20                  25                  30
   Leu Arg Pro Glu His Phe Gln Glu Val Gly Tyr Ala Ala Pro Pro Ser
           35                  40                  45
   Pro Pro Leu Ser Arg Ser Leu Pro Met Asp His Pro Asp Ser Ser Gln
       50                  55                  60
   His Gly Pro Pro Phe Glu Gly Gln Ser Gln Val Gln Pro Leu Pro Leu
   65                  70                  75                  80
   Arg Arg Pro Pro Leu Phe Gln Gln Glu Lys Leu Leu Pro Ala Gln Leu
                   85                  90                  95
   Pro Ala Glu Lys Glu Val Gly Pro Pro Leu Pro Gln Glu Ala Val Pro
               100                 105                 110
   Leu Gln Lys Glu Leu Pro Ser Leu Gln His Pro Asn Glu Gln Lys Glu
           115                 120                 125
   Gly Thr Pro Ala Pro Phe Gly Asp Gln Ser His Pro Glu Pro Glu Ser
       130                 135                 140
   Trp Asn Ala Ala Gln His Cys Gln Gln Asp Arg Ser Gln Gly Gly Trp
   145                 150                 155                 160
   Gly His Arg Leu Asp Gly Phe Pro Pro Gly Arg Pro Ser Pro Asp Asn
                   165                 170                 175
   Leu Asn Gln Ile Cys Leu Pro Asn Arg Gln His Val Val Tyr Gly Pro
               180                 185                 190
   Trp Asn Leu Pro Gln Ser Ser Tyr Ser His Leu Thr Arg Gln Gly Glu
           195                 200                 205
   Thr Leu Asn Phe Leu Glu Ile Gly Tyr Ser Arg Cys Cys His Cys Arg
       210                 215                 220
   Arg His Thr Asn Arg Leu Glu Cys Ala Lys Leu Val Trp Glu Glu Ala
   225                 230                 235                 240
   Met Ser Arg Phe Cys Glu Ala Glu Phe Ser Val Lys Thr Arg Pro His
                   245                 250                 255
   Trp Cys Cys Thr Arg Gln Gly Glu Ala Arg Phe Ser Cys Phe Gln Glu
               260                 265                 270
   Glu Ala Pro Gln Pro His Tyr Gln Leu Arg Ala Cys Pro Ser His Gln
           275                 280                 285
   Pro Asp Ile Ser Ser Gly Leu Glu Leu Pro Phe Pro Pro Gly Val Pro
       290                 295                 300
   His Trp Thr Ile Ser Arg Thr Ser Cys His Leu Arg Arg Phe Arg Ser
   305                 310                 315                     320
   Val Pro Arg Asn Leu Pro Ala Thr Asp Pro Leu Gln Arg Glu Leu Leu
                   325                 330                 335
   Ala Leu Ile Gln Leu Glu Arg Glu Phe Gln Arg Cys Cys Arg Gln Gly
               340                 345                 350
   Asn Asn His Thr Cys Thr Trp Lys Ala Trp Glu Asp Thr Leu Asp Lys
           355                 360                 365
   Tyr Cys Asp Arg Glu Tyr Ala Val Lys Thr His His His Leu Cys Trp
       370                 375                 380
   Arg His Pro Pro Ser Pro Thr Arg Asp Glu Cys Phe Gly Arg Arg Ala
   385                 390                 395                     400
   Pro Tyr Pro Asn Tyr Asp Arg Asp Ile Leu Thr Ile Asp Ile Gly Arg
                   405                 410                 415
   Val Thr Pro Asn Leu Met Gly His Leu Cys Gly Asn Gln Arg Val Leu
               420                 425                 430
   Thr Lys His Lys His Ile Pro Gly Leu Ile His Asn Met Thr Ala Arg
           435                 440                 445
   Cys Cys Asp Leu Pro Phe Pro Glu Gln Ala Cys Cys Ala Glu Glu Glu
       450                 455                 460
   Lys Leu Thr Phe Ile Asn Asp Leu Cys Gly Pro Arg Arg Asn Ile Trp
   465                 470                 475                     480
   Arg Asp Pro Ala Leu Cys Cys Tyr Leu Ser Pro Gly Asp Glu Gln Val
                   485                 490                 495
   Asn Cys Phe Asn Ile Asn Tyr Leu Arg Asn Val Ala Leu Val Ser Gly
               500                 505                 510
   Asp Thr Glu Asn Ala Lys Gly Gln Gly Glu Gln Gly Ser Thr Gly Gly
           515                 520                 525
   Thr Asn Ile Ser Ser Thr Ser Glu Pro Lys Glu Glu
       530                 535                 540
(2) INFORMATION FOR SEQ ID NO:3:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   CGGGATCCGC CATCATGGGG ACCACAGCCA G 31
(2) INFORMATION FOR SEQ ID NO:4:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   GCTCTAGATC CAAGAGGTGT TTAGTG 26
(2) INFORMATION FOR SEQ ID NO:5:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GCGCGGATCC ACCATGGGGA CCACAGCCAG A 31
(2) INFORMATION FOR SEQ ID NO:6:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
   GCGCTCTAGA TCAAGCGTAG TCTGGGACGT CGTATGGGTA TTCTTCCTTG GGCTC 55
(2) INFORMATION FOR SEQ ID NO:7:
   (i) SEQUENCE CHARACTERISTICS:
      (A) LENGTH: 559 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:7:
   Met Gly Thr Val Ser Arg Ala Ala Leu Ile Leu Ala Cys Leu Ala Leu
   1               5                   10                  15
   Ala Ser Ala Ala Ser Glu Gly Ala Phe Lys Ala Ser Asp Gln Arg Glu
   20 25 30
   Met Thr Pro Glu Arg Leu Phe Gln His Leu His Glu Val Gly Tyr Ala
   35 40 45
   Ala Pro Pro Ser Leu Pro Gln Thr Arg Arg Leu Arg Val Asp His Ser
   50 55 60
   Val Thr Ser Leu His Asp Pro Pro Leu Phe Glu Glu Gln Arg Glu Val
   65 70 75 80
   Gln Pro Pro Ser Ser Pro Glu Asp Ile Pro Val Tyr Glu Glu Asp Trp
   85 90 95
   Pro Thr Phe Leu Asn Pro Asn Val Asp Lys Ala Gly Pro Ala Val Pro
   100 105 110
   Gln Glu Ala Ile Pro Leu Gln Lys Glu Gln Pro Pro Pro Gln Val His
   115 120 125
   Ile Glu Gln Lys Glu Ile Asp Pro Pro Ala Gln Pro Gln Glu Glu Ile
   130 135 140
   Val Gln Lys Glu Val Lys Pro His Thr Leu Ala Gly Gln Leu Pro Pro
   145 150 155 160
   Glu Pro Arg Thr Trp Asn Pro Ala Arg His Cys Gln Gln Gly Arg Arg
   165 170 175
   Gly Val Trp Gly His Arg Leu Asp Gly Phe Pro Pro Gly Arg Pro Ser
   180 185 190
   Pro Asp Asn Leu Lys Gln Ile Cys Leu Pro Glu Arg Gln His Val Ile
   195 200 205
   Tyr Gly Pro Trp Asn Leu Pro Gln Thr Gly Tyr Ser His Leu Ser Arg
   210 215 220
   Gln Gly Glu Thr Leu Asn Val Leu Glu Thr Gly Tyr Ser Arg Cys Cys
   225 230 235 240
   Pro Cys Arg Ser Asp Thr Asn Arg Leu Asp Cys Leu Lys Leu Val Trp
   245 250 255
   Glu Asp Ala Met Thr Gln Phe Cys Glu Ala Glu Phe Ser Val Lys Thr
   260 265 270
   Arg Pro His Leu Cys Cys Arg Leu Arg Gly Glu Glu Arg Phe Ser Cys
   275 280 285
   Phe Gln Lys Glu Ala Pro Arg Pro Asp Tyr Leu Leu Arg Pro Cys Pro
   290 295 300
   Val His Gln Asn Gly Met Ser Ser Gly Pro Gln Leu Pro Phe Pro Pro
   305 310 315 320
   Gly Leu Pro Thr Pro Asp Asn Val Lys Asn Ile Cys Leu Leu Arg Arg
   325 330 335
   Phe Arg Ala Val Pro Arg Asn Leu Pro Ala Thr Asp Ala Ile Gln Arg
   340 345 350
   Gln Leu Gln Ala Leu Thr Arg Leu Glu Thr Glu Phe Gln Arg Cys Cys
   355 360 365
   Arg Gln Gly His Asn His Thr Cys Thr Trp Lys Ala Trp Glu Gly Thr
   370 375 380
   Leu Asp Gly Tyr Cys Glu Arg Glu Leu Ala Ile Lys Thr His Pro His
   385 390 395 400
   Ser Cys Cys His Tyr Pro Pro Ser Pro Ala Arg Asp Glu Cys Phe Ala
   405 410 415
   His Leu Ala Pro Tyr Pro Asn Tyr Asp Arg Asp Ile Leu Thr Leu Asp
   420 425 430
   Leu Ser Arg Val Thr Pro Asn Leu Met Gly Gln Leu Cys Gly Ser Gly
   435 440 445
   Arg Val Leu Ser Lys His Lys Gln Ile Pro Gly Leu Ile Gln Asn Met
   450 455 460
   Thr Val Arg Cys Cys Glu Leu Pro Tyr Pro Glu Gln Ala Cys Cys Gly
   465 470 475 480
   Glu Glu Glu Lys Leu Ala Phe Ile Glu Asn Leu Cys Gly Pro Arg Arg
   485 490 495
   Asn Ser Trp Lys Asp Pro Ala Leu Cys Cys Asp Leu Ser Pro Glu Asp
   500 505 510
   Lys Gln Ile Asn Cys Phe Asn Thr Asn Tyr Leu Arg Asn Val Ala Leu
   515 520 525
   Val Ala Gly Asp Thr Gly Asn Ala Thr Gly Leu Gly Glu Gln Gly Pro
   530 535 540
   Thr Arg Gly Thr Asp Ala Asn Pro Ala Pro Gly Ser Lys Glu Glu
   545 550 555